**Europäisches Patentamt**

**European Patent Office**

**Office européen des brevets**

(19)

(11) Veröffentlichungsnummer : **0 043 986**
**B1**

(12)     **EUROPÄISCHE PATENTSCHRIFT**

(45) Veröffentlichungstag der Patentschrift :
**13.03.85**

(21) Anmeldenummer : **81105092.1**

(22) Anmeldetag : **01.07.81**

(51) Int. Cl.⁴ : **C 07 C 41/06,** C 07 C 43/04,
B 01 J 31/08

(54) **Verfahren zur Herstellung von Alkyl-tert.-alkylethern.**

(30) Priorität : **12.07.80 DE 3026504**

(43) Veröffentlichungstag der Anmeldung :
**20.01.82 Patentblatt 82/03**

(45) Bekanntmachung des Hinweises auf die Patenterteilung : **13.03.85 Patentblatt 85/11**

(84) Benannte Vertragsstaaten :
**BE DE FR GB NL**

(56) Entgegenhaltungen :
**DE-A- 2 629 769**
**DE-C-   906 453**
**FR-A- 1 583 594**

(73) Patentinhaber : **EC ERDÖLCHEMIE GMBH**
**Postfach 75 20 02**
**D-5000 Köln 71 (DE)**

(72) Erfinder : **Köhler, Hans-Dieter, Dr.**
**Stürzelberger Strasse 71**
**D-4047 Dormagen 5 (DE)**
Erfinder : **Schleppinghoff, Bernard, Dr.**
**Adolf-Kolping-Strasse 5**
**D-4047 Dormagen 1 (DE)**

(74) Vertreter : **Mann, Volker, Dr. et al**
**c/o Bayer Aktiengesellschaft Zentralbereich Patente**
**Marken und Lizenzen**
**D-5090 Leverkusen-Bayerwerk (DE)**

## Beschreibung

Die vorliegende Erfindung betrifft ein Verfahren zur Herstellung von Alkyl-tert.-alkylethern aus an der Doppelbindung verzweigten Isoolefinen und Alkanolen in Gegenwart von Kationenaustauschern, die mit einem Metall der VI., VII. oder VIII. Nebengruppe des Periodensystems in elementarer Form belegt sind.

Es ist bekannt, daß Methyl-tert.-butylether ein klopffester Vergaserkraftstoff-Zusatz ist, der zusätzlich in einem gewissen Ausmaß die Kohlenmonoxid-Konzentration im Abgas eines Verbrennungsmotors herabsetzt (DE-OS 24 19 439). Es ist bereits bekannt, Olefine, gegebenenfalls im Gemisch mit Paraffinkohlenwasserstoff, mit primären oder sekundären Alkoholen in Gegenwart von sauren Katalysatoren zu Alkylethern umzusetzen. Als saure Katalysatoren werden beispielsweise anorganische Säuren wie Schwefelsäure, oder organischen Säuren wie p-Toluolsulfonsäuren oder saure, sulfonsäuregruppenhaltige Kationenaustauscher auf der Basis vernetzter vinylaromatischer Polymerer verwendet (DE-AS 12 24 294). Die Reaktion zwischen einem Olefin und einem Alkohol zu dem korrespondierenden Ether hat einen Gleichgewichtspunkt, welcher für die Synthese des Ethers umso günstiger liegt, je niedriger die Temperatur des Reaktion ist, wobei aber gleichzeitig die Reaktionsgeschwindigkeit abnimmt (DE-OS 25 21 964). Zur Verbesserung der Umwandlungsrate bei im übrigen unveränderten Reaktionsbedingungen ist vorgeschlagen worden, einen Teil des Reaktionsgemisches aus dem Olefin mit einem primären Alkohol direkt in das Einsatzprodukt für den Herstellungsreaktor zurückzuführen (DE-OS 29 11 077). Ein höherer Olefinumsatz zur besseren Ausnutzung dieses Rohstoffes kann auch durch den Einsatz eines Alkoholüberschusses erreicht werden, wobei die spätere Abtrennung des überschüssigen Alkohols durch Extraktion mit Wasser (DE-OS 24 19 439) oder durch Extraktivdestillation mit polaren Lösungsmitteln (japanische Patentanmeldung 7300509) erheblichen zusätzlichen Aufwand erfordert. Verfahren, bei denen zur Steigerung der Olefinumsätze Rückführungen der nichtumgesetzten Olefine in direkter oder indirekter Form, wie nach Durchlaufen von physikalischen Trennoperationen, vorgesehen sind, steigern zwar die Ausbeute der eingesetzten Olefine, bedingen aber einen höheren Aufwand an Investitions- und Energiekosten für größere Reaktionsgefäße und die erforderlichen Trenneinrichtungen.

Es ist weiterhin bekannt, die Umsetzung von Methanol und Isobutylen zu Methyl-tert.-butylether mit Rutheniumtrichlorid zu katalysieren (US 3 718 701).

Es ist weiterhin bekannt, daß sich Olefine in Gegenwart von sauren Kationenaustauschern zu dimeren, trimeren und höheren Oligomeren umwandeln lassen (Erdöl und Kohle *19*, 497 (1966)).

Es wurde nun ein Verfahren zur Herstellung von Alkyl-tert.-alkylethern aus an der Doppelbindung verzweigten Isoolefinen und Alkanolen in Gegenwart von Kationenaustauschern gefunden, das dadurch gekennzeichnet ist, daß man zur Veretherung makroporöse oder gelförmige, saure Kationenaustauscher in der H$^+$-Form, die mit 0,1 bis 5 g eines Metalls der VI., VII. oder VIII. Nebengruppe des Periodensystems der Elemente (Mendelejew) in elementarer Form pro 1 Liter trockenen Kationenaustauscher belegt worden sind, benutzt, welche Vernetzungsgrade von 2 bis 65 % und eine spezifische Oberfläche von 5 bis 750 m$^2$ pro g trockenes Austauscherharz aufweisen, wobei das gewünschte Metall in Form eines nicht komplexen, kationischen Salzes mit dem Kationenaustauscher zusammengebracht wird und der Kationenaustauscher nach Neutralwaschen und Trocknen in einer Wasserstoffatmosphäre bei einem Druck von 2-50 bar und einer Temperatur von 50-140 °C behandelt wird, und daß die Veretherungsreaktion in an sich bekannter Weise in der flüssigen Phase bei 30 bis 140 °C, einem Druck von 2 bis 100 bar und einem Molverhältnis von Isoolefin zu Alkanol wie 0,1 bis 5, gegebenenfalls in Gegenwart eines Lösungsmittels, durchgeführt wird.

Für das erfindungsgemäße Verfahren werden makropöse oder gelförmige, saure Kationenaustauscher eingesetzt, die beispielsweise durch Copolymerisation von Vinylmonomeren mit Divinylvernetzern in Gegenwart von Lösungsmitteln, die die Monomeren lösen aber keine Quellwirkung für die entstehenden Polymeren zeigen, hergestellt werden (DE-AS 11 13 570, US 3 586 646). Als Vinylmonomere seien u. a. Styrol oder Acrylsäureester genannt. Als Divinylvernetzer kommt beispielsweise Di-vinylbenzol in Frage. Der Vernetzungsgrad ist abhängig von der Menge des Divinylvernetzers und kann beispielsweise von 2 bis 65 %, bevorzugt von 8 bis 25 %, schwanken, wobei diese Zahlenangaben die Menge des Vernetzers, bezogen auf die Gesamtmenge an Comonomeren, bedeuten.

Als saure Gruppen kommen beispielsweise Carboxylgruppen, die durch Verseifung von Acrylsäureester erhalten werden, oder Sulfonsäuregruppen, die durch nachträgliche Sulfonierung eingeführt werden können, in Betracht.

Bevorzugt werden stark saure, sulfonsäuregruppenhaltige Styrol-Divinylbenzol-Polymerisate für das erfindungsgemäße Verfahren eingesetzt. Solche Kationenaustauscher sind beispielsweise unter der Bezeichnung LEWATIT SPC 118, LEWATIT SPC 120, Amberlite 200 C, Dowex MSC-1 oder Duolite C 26 handelsüblich.

Die erfindungsgemäß einsetzbaren Kationenaustauscher weisen eine spezifische Oberfläche von beispielsweise 5 bis 750 m$^2$ pro g, bevorzugt 50 bis 250 m$^2$ pro g, auf. Der mittlere Porenradius kann in den Grenzen von 50 bis 1 200 Å, bevorzugt von 70 bis 500 Å, variieren. Beim Einsatz der Kationenaustauscher als Perlpolymerisate werden Korngrößen von beispielsweise 0,1 bis 2 mm verwendet. Beim Einsatz der Kationenaustauscher als Pulverharze können beispielsweise Kornverteilungen von 10 bis 100 μ zum Einsatz kommen.

2

Im erfindungsgemäßen Verfahren werden nun solche Kationenaustauscher in der H+-Form verwendet, die mit 0,1 bis 5 g, bevorzugt 0,2 bis 3 g, eines Metalls der VI., VII. oder VIII. Nebengruppe des Periodensystems des Elemente in elementarer Form pro Liter trockenem Kationenaustauscher belegt worden sind. Als Metalle der VI., VII. oder VIII. Nebengruppe des Periodensystems des Elemente (Mendelejew) seien beispielsweise genannt : Chrom, Molybdän, Wolfram, Mangan, Technetium, Rhenium, Eisen, Kobalt, Nickel, Ruthenium, Rhodium, Palladium, Osmium, Iridium, Platin, bevorzugt Chrom, Rhenium, Palladium, Platin oder Eisen.

Zur Belegung der Kationenaustauscher mit den genannten Metallen wird das gewünschte Metall in Form eines nicht-komplexen, kationischen Salzes ansatzweise mit dem Kationenaustauscher in der H+-Form in an sich bekannter Weise zusammengebracht. Als solche Salze seien beispielsweise die Chloride, Bromide, Nitrate, Sulfate oder Acetate genannt. Die Menge des einzusetzenden Salzes wird so bemessen, daß das elementare Metall in einer Menge von 0,1 bis 5 g, bevorzugt 0,2 bis 3 g, pro Liter Austauscher vorliegt, und kann durch den Fachmann durch einfaches Probieren ermittelt werden. Es kann vorteilhaft sein, die beim Umsatz des Kationenaustauschers mit dem Metallsalz entstehende Säure zu neutralisieren. Diese Neutralisation kann während oder nach dem Umsatz des Kationenaustauschers mit dem Salz vorgenommen werden. Hierzu kann eine alkalisch reagierende Verbindung, gegebenenfalls in Form ihrer wäßrigen Lösung, wie Natriumhydroxid, Kaliumhydroxid, Ammoniumhydroxid, Natriumcarbonat, Kaliumcarbonat oder Ammoniumcarbonat, verwendet werden. Der Kationenaustauscher wird nach dem Umsatz mit dem Metallsalz neutral gewaschen und anschließend bei höherer Temperatur, gegebenenfalls unter vermindertem Druck, getrocknet. Beispielsweise sei eine Trocknung bei 100 °C und Wasserstrahlpumpenvakuum für 24 Stunden genannt.

Zur Überführung der auf dem Kationenaustauscher fixierten Ionen eines Metalls der VI., VII. oder VIII. Nebengruppe des Periodensystems (Mendelejew) in die elementare Form wird der mit den Metallionen belegte und getrocknete Kationenaustauscher in Wasserstoff-Atmosphäre bei einem Druck von 2 bis 50 bar, bevorzugt 20 bis 30 bar, und einer Temperatur von 50 bis 140 °C, bevorzugt 80 bis 120 °C, behandelt.

Die erfindungsgemäße Umsetzung der verzweigten Isoolefine mit den Alkanolen in Gegenwart des mit elementaren Metallen belegten Kationenaustauschers kann in der flüssigen oder der gasförmigen Phase, bevorzugt in der flüssigen Phase, durchgeführt werden. Als Temperatur für diese Umsetzung sei beispielsweise eine Temperatur von 30 bis 140 °C, bevorzugt 50 bis 130 °C, besonders bevorzugt 70 bis 120 °C, ganz besonders bevorzugt 75° bis 110 °C, genannt. Die Umsetzung wird bei einem Druck von 2 bis 100 bar, bevorzugt 3 bis 30 bar, durchgeführt. Für den Fall, daß in bevorzugter Weise in der Flüssigphase gearbeitet werden soll, werden im Rahmen der genannten Temperaturen und Drucke stets solche Kombinationen gewählt, bei denen zumindest ein Teil des Reaktionsproduktes in der Flüssigphase vorliegt.

Im erfindungsgemäßen Verfahren werden an der Doppelbindung verzweigte Isoolefine eingesetzt. Beispielhaft seien solche Isoolefine mit 4 bis 12, bevorzugt 4 bis 8, besonders bevorzugt 4 bis 6, Kohlenstoffatomen genannt, wie Isobutylen, Methylbutene, Methylpentene, Dimethylpentene, Methylhexene, Dimethylhexene.

Solche Olefine können in reiner Form oder in Form eines Gemisches aus einem solchen Olefin und gesättigten Kohlenwasserstoffen eingesetzt werden. Beispielsweise kann eine in einer Raffinerie aus Crackprozessen anfallende Fraktion im erfindungsgemäßen Verfahren eingesetzt werden, die eines der o. g., an der Doppelbindung verzweigten Isoolefine neben gesättigten Kohlenwasserstoffen und gegebenenfalls inerten Gasen, wie Stickstoff, Wasserstoff oder Kohlendioxid enthalten. Weiterhin ist es möglich, Gemische oder Crackfraktionen im erfindungsgemäßen Verfahren einzusetzen, die 2 oder mehr an der Doppelbindung verzweigte Isoolefine, gegebenenfalls neben Paraffinen und/oder den genannten Inertstoffen enthalten, und die nach Durchführung der erfindungsgemäßen Reaktion erhaltenen Alkyl-tert.-alkylether durch geeignete Maßnahmen, beispielsweise durch Destillation, zu trennen.

Als Alkanole, die im erfindungsgemäßen Verfahren eingesetzt werden können, seien beispielsweise solche mit 1 bis 8, bevorzugt 1 bis 6, besonders bevorzugt 1 bis 4, Kohlenstoffatomen genannt, wie Methanol, Ethanol, Propanol, Butanole, Hexanole oder Oktanole.

Das an der Doppelbindung verzweigte Isoolefin wird in einem Molverhältnis zu dem Alkanol wie 0,1 bis 5, bevorzugt 0,5 bis 2, besonders bevorzugt 0,9 bis 1,1, eingesetzt.

Das erfindungsgemäße Verfahren kann sowohl kontinuierlich als auch diskontinuierlich durchgeführt werden.

In der diskontinuierlichen Variante kann das Verfahren beispielsweise wie folgt durchgeführt werden :

In einem geschlossenen Reaktor wird das zu verethernde Isoolefin, gegebenenfalls im Gemisch mit einem oder mehreren weiteren Isoolefinen und/oder inerten Kohlenwasserstoffen mit Methanol im etwa molaren Verhältnis, bezogen auf die Isoolefine, und mit dem oben beschriebenen Austauscherharz im Verhältnis 10 Gewichtsteile Reaktionsgemisch pro 1 Gewichtsteil Austauscherharz versetzt und etwa eine halbe bis 5 Stunden unter Druck bei erhöhter Temperatur gerührt oder geschüttelt, bis die Veretherung abgeschlossen ist. Danach kann das Reaktionsgemisch entspannt werden und vom Austauscherharz abdekantiert werden. Das Reaktionsgefäß wurde so unter Wiederverwendung des Austauscherharzes mit einem neuen Reaktionsansatz 15 mal beschickt, ohne eine wesentliche Kontaktaktivitätsabnahme festzustellen.

In der Variante des kontinuierlichen Verfahrens kann beispielsweise wie folgt verfahren werden :

Der oben beschriebene Kationenaustauscher wird in ein temperierbares Edelstahl- oder Glasgefäß, beispielsweise 1 oder mehrere temperierbare Reaktionsrohre, eingefüllt. Das Isoolefin oder eines seiner oben beschriebenen Gemische und das Alkanol werden einzeln oder als Gemisch von unten nach oben oder umgekehrt, beispielsweise in der Rieselphase, durch das von außen temperierbare Austauscherbett geführt. Bevorzugt wird hierbei mit einer konstanten Fließgeschwindigkeit gearbeitet. Durch Variation des Druckes, der Temperatur und der Kontaktbelastung bzw. der Verweilzeit können durch einfaches Ausprobieren die für die verschiedenen Isoolefine oder ihre Gemische günstigsten Bedingungen gefunden werden. Der Reaktorausfluß wird gesammelt und der Gehalt an Ether darin beispielsweise durch gaschromatographische Analyse bestimmt. Als Kontaktbelastung a wird hierbei das Verhältnis der Menge Kohlenwasserstoffgemisch in ml zur Menge Ionenaustauscherkontakt in ml pro Zeit in Stunden definiert. Im erfindungsgemäßen Verfahren können Kontaktbelastungen von a = 0,1 bis a = 10 eingestellt werden.

Ein Teil des Reaktionsgemisches kann aus dem Reaktor direkt zurückgeführt werden, wobei das Gewichtsverhältnis vom Rückfuhrstrom zu Ausspeisstrom 0,1 bis 10 : 1, bevorzugt 1 : 1 bis 2 : 1 beträgt.

Das erfindungsgemäße Verfahren zeichnet sich dadurch aus, daß Isoolefine und Alkanole in einem Reaktordurchgang bei technisch nutzbaren Raumgeschwindigkeiten fast quantitativ zum entsprechenden Ether umgesetzt werden.

Es ist überraschend, daß die erfindungsgemäße Veretherung bei höheren Temperaturen und damit höheren Raum-Zeit-Ausbeuten durchgeführt werden kann, ohne daß eine merkbare Olefinoligomerisierung abläuft, wie sie nach dem Stande der Technik in Gegenwart von sauren Kationenaustauschern zu erwarten wäre.

## Beispiele

Der in den Beispielen verwendete Begriff Kontaktbelastung a ist durch den folgenden Quotienten definiert :

$$a = \text{[Menge Kohlenwassergemisch (ml)]}/\text{[Menge Kontakt (ml) . Zeit (Stdn.)]}$$

## Beispiel 1

Entsprechend DE-PS 1 113 570, Beispiel 3, wurde ein makroporöser Kationenaustauscher hergestellt. Der wasserfeuchten $H^+$-Form dieses Kationenaustauschers wird im batch soviel Palladiumacetat angeboten, daß 0,75 g Pd/1 Liter trockenes Harz nach Reduktion mit Wasserstoff auf dem Kationenaustauscher sind. Die bei der Behandlung mit Palladiumacetat freigesetzte Säure wird mit 1 gew.-%iger NaOH neutralisiert. Anschließend wird der neutral gewaschene Kationenaustauscher 24 Stunden bei 100 °C im Wasserstrahlpumpenvakuum getrocknet.

Die Aktivierung des Palladiums auf dem Kationenaustauscher erfolgt zwischen 90 und 100 °C und 20 bis 25 bar $H_2$ innerhalb von 48 Stunden.

## Beispiel 2

Als Veretherungsapparatur fand ein temperierbarer Durchlaufreaktor Anwendung. Bei einem vorgegebenen lichten Reaktordurchmesser von 20 mm wurde die Katalysatorbetthöhe jeweils so gewählt, daß das daraus resultierende Reaktionsvolumen zusammen mit der eingesetzten Flüssigkeitsmenge die gewünschte kontaktbelastung a ergab. Zur Temperaturkontrolle is der Reaktor mit mehreren Temperaturmessungen in Abständen von je 100 mm ausgerüstet, wobei die Führungstemperatur jeweils frei gewählt werden kann. Der Reaktor ist über eine Druckhaltung druckgeregelt. Die Abmischung der Kohlenwasserstoffe mit dem Alkanol erfolgt in einer Mischkammer vor dem Reaktor. Das molare Verhältnis aktive Kohlenwasserstoffe zu Alkanol beträgt 1 : 1. Die Reaktoreinsatzprodukte können wahlweise temperiert, die Teilströme pumpengesteuert werden. Der Reaktor ist temperierbar. Das Reaktorausgangsprodukt wird in einem gekühlten Abscheider zwischengelagert, bevor es in einer sich anschließenden Destillationskolonne aufgearbeitet wird. Der Tertiäralkyl-alkylether läßt sich hierbei als Seitenstrom abnehmen. Das Kopfprodukt, welches noch aktive Komponenten wie Methylbutene enthält, kann zum Reaktoreingang zurückgeführt werden. Das Kolonnensumpfprodukt enthält höhere Ether. Die Analysen erfolgen gaschromatographisch.

In einer typischen Ausführung wird der wie im Beispiel 1 beschriebene Kationenaustauscher im Wasserstoffstrom bei 90 °C, 26 bar $H_2$ für 48 Stunden in der Veretherungsapparatur aktiviert. Danach erfolgt die stufenweise Zudosierung des Reaktionsgemisches so lange, bis sich eine Kontaktbelastung a = 1 (ml/ml.h) eingestellt hat. Die Veretherung aktiver Methylbutene mit Methanol im äquimolaren Verhältnis zu Tertiäramyl-methylether (TAME) verläuft bei einer Reaktionstemperatur von 110 °C und einem Reaktionsdruck von 8 bar in der Rieselphase. Die Probenahme zur gaschromatographischen Bestimmung der Endprodukte erfolgt nach 60 Stunden konstanter Kontaktbelastung. Die Zusammensetzung des Einsatz- und Endproduktes ist in Tabelle 1 zusammengestellt :

Tabelle 1

| | | Ausgangs-produkt | Endprodukt |
|---|---|---|---|
| 2-Methylbuten-(2) | (g) | 14,5 | 1,6 |
| 2-Methylbuten-(1) | (g) | 3,8 | 0,2 |
| 3-Methylbuten-(1) | (g) | 0,5 | $<0,1$ |
| Methanol | (g) | 9,5 | 0,9 |
| TAME | (g) | – | 24,7 |
| Ausbeute an TAME | (%) | – | 90,1 |

Die Ergänzung zu 100 Gew.-% bilden höhere, durch IR-Spektroskopie als Ether identifizierte Verbindungen $C_5$-, $C_6$- und höhere Olefine und Paraffine.

Beispiel 3 (Vergleichsbeispiel)

Entsprechend der deutschen Patenschrift 1 113 570, Beispiel 3, wurde ein makroporöser Kationenaustauscher hergestellt. Der so erhaltene wasserfeuchte Ionenaustauscher wurde zur Entfernung des Wassers im Trockenharz nach der herkömmlichen Trocknungsmethode bei 90 bis 100 °C, 15 Torr über Nacht getrocknet. Mit dem so erhaltenen trockenen Ionenaustauscher wurde wie im Beispiel 2 verfahren, ohne die partielle Belegung mit Palladium und dessen Reduktion in den metallischen Zustand durchzuführen. Die Zusammensetzung des Einsatz- und Endproduktes ergibt sich aus Tabelle 2 :

Tabelle 2

| 110°C/8 bar | | Ausgangs-produkt | End-produkt |
|---|---|---|---|
| 2-Methylbuten-(2) | (g) | 14,5 | 3,4 |
| 2-Methylbuten-(1) | (g) | 3,8 | 2,2 |
| 3-Methylbuten-(1) | (g) | 0,5 | 0,4 |
| Methanol | (g) | 9,5 | 4,0 |
| TAME | (g) | – | 17,6 |
| Ausbeute an TAME | (%) | – | 64 |

Beispiel 4

Die Versuche wurden in gleicher Weise wie in Beispiel 2 durchgeführt, jedoch unter Verwendung des LEWATIT SPC 118 der Bayer AG mit 1,25 g Palladium/l getrockneten Kontakt nach Reduktion mit Wasserstoff. Die Ergebnisse in Tabelle 3 geben den Einfluß des Druckes auf die Veretherung wieder :

Tabelle 3

| 110°C/a = 1 | | Einsatz-produkt | Endprodukt | | | |
|---|---|---|---|---|---|---|
| | | | 3 bar | 10 bar | 15 bar | 25 bar |
| 2-Methylbuten-(2) | (g) | 14,5 | 6,8 | 1,8 | 1,6 | 1,7 |
| 2-Methylbuten-(1) | (g) | 3,8 | 0,5 | 0,5 | 0,7 | 0,6 |
| 3-Methylbuten-(1) | (g) | 0,5 | 0,5 | $<0,1$ | 0,2 | 0,2 |
| Methanol | (g) | 9,5 | 5,4 | 1,1 | 1,2 | 1,2 |
| TAME | (g) | – | 16,1 | 24,1 | 23,9 | 23,8 |
| Ausbeute an TAME | (%) | – | 58,5 | 87,8 | 87,2 | 86,7 |

Die Tabelle 3 zeigt, daß bei 110 °C und einer Kontaktbelastung a = 1 bei einem Druck, der die Aufrechterhaltung einer Flüssigphase gewährleistet und der bei 110 °C oberhalb von etwa 8 bar liegt, die TAME-Ausbeute ziemlich konstant ist.

Beispiel 5

Die Versuche wurden in gleicher Weise wie in Beispiel 2 durchgeführt, jedoch unter Verwendung des LEWATIT SPC 120 der Bayer AG mit 2 g Palladium/l getrocknetem Kontakt nach Reduktion mit Wasserstoff. Die Zusammensetzung des Einsatz- und Endproduktes, die sich bei verschiedenen Reaktionstemperaturen ergab, ist in Tabelle 4 zusammengestellt :

Tabelle 4

| 6 bar/a = 1 | | Einsatz-produkt | Endprodukt | | | |
|---|---|---|---|---|---|---|
| | | | 70° | 90° | 100° | 110° |
| 2-Methylbuten-(2) | (g) | 14,5 | 3,2 | 2,0 | 1,6 | 1,6 |
| 2-Methylbuten-(1) | (g) | 3,8 | 1,7 | 0,5 | 0,4 | 0,3 |
| 3-Methylbuten-(1) | (g) | 0,5 | 0,3 | 0,1 | < 0,1 | < 0,1 |
| Methanol | (g) | 9,5 | 2,5 | 1,3 | 0,9 | 0,9 |
| TAME | (g) | – | 19,9 | 23,7 | 24,5 | 24,7 |
| Ausbeute an TAME | (%) | – | 72,4 | 86,2 | 89,4 | 90,1 |

Aus den in Tabelle 4 aufgeführten Ergebnissen ergibt sich, daß bei etwa 75 bis 110 °C die besten TAME-Ausbeuten erzielt wurden.

Beispiel 6

Das Einsatzmaterial wurde im Aufwärtsstrom mit unterschiedlicher Kontaktbelastung a durch das Katalysatorbett geleitet, wobei die Probeentnahme jeweils nach 24 Stunden konstanter Kontaktbelastung erfolgte. Als Kontakte dienten wieder die in Beispiel 2 beschriebenen. Die Ergebnisse für verschiedene Kontaktbelastungen sind in Tabelle 5 angeführt :

Tabelle 5

| 80°/10 bar | | Einsatz-produkt | Endprodukt | | |
|---|---|---|---|---|---|
| | | | a = 0,5 | a = 1,0 | a = 1,5 |
| 2-Methylbuten-(2) | (g) | 14,5 | 1,2 | 2,3 | 2,6 |
| 2-Methylbuten-(1) | (g) | 3,8 | 0,2 | 0,3 | 0,6 |
| 3-Methylbuten-(1) | (g) | 0,5 | < 0,1 | < 0,1 | < 0,1 |
| Methanol | (g) | 9,5 | 1,4 | 2,1 | 2,4 |
| TAME | (g) | – | 25,5 | 23,6 | 22,7 |
| Ausbeute an TAME | (%) | – | 92,5 | 86,0 | 83 |

Beispiel 7

Die Versuche wurden in gleicher Weise und mit dem gleichen Katalysator wie in Beispiel 2 beschrieben durchgeführt. Das Einsatzmaterial wurde jedoch mit Ethanol an Stelle von Methanol zum Ethyltertiäramylether umgesetzt.

6

**0 043 986**

Tabelle 6

| 90°C/8 bar/a = 1 | | Einsatz-produkt | Endprodukt |
|---|---|---|---|
| 2-Methylbuten-(2) | (g) | 13,7 | 1,5 |
| 2-Methylbuten-(1) | (g) | 2,6 | 0,3 |
| 3-Methylbuten-(1) | (g) | 0,5 | - |
| Ethanol | (g) | 13,1 | 1,1 |
| Ether | (g) | - | 30,1 |
| Ausbeute an Ether | (%) | - | 91 |

Beispiel 8

Die Versuche wurden in gleicher Weise und mit dem gleichen Katalysator wie in Beispiel 2 beschrieben durchgeführt. Als Einsatzmaterial wurde ein $C_4$-Kohlenwasserstoff-Schnitt, der neben 44,1 Gew.-% Isobutylen noch $C_4$-Olefine und Paraffine enthält, mit Methanol verethert.

Tabelle 7

| 12 bar/a = 1 | | Einsatz-produkt | Endprodukt 40°C | 45°C |
|---|---|---|---|---|
| Isobutylen | (g) | 44,1 | 1,3 | 1,2 |
| Methanol | (g) | 25,3 | 0,3 | 0,4 |
| Methyltertiär-butylether (MTBE) | (g) | - | 63,3 | 63,8 |
| Isobutylenumsatz | (%) | - | 97,1 | 97,3 |
| MTBE-Ausbeute | (%) | - | 91,3 | 92,0 |

Beispiel 9

Die Versuche wurden in gleicher Weise wie in Beispiel 2 durchgeführt, jedoch unter Verwendung des nach Beispiel 1 hergestellten makroporösen Kationenaustauschers, der mit den in Tabelle 8 angegebenen, mit Wasserstoff reduzierten Metallen dotiert worden war.

Tabelle 8

| 90°/8 bar | | Ausgangsprodukt | Endprodukt Chrom 1,0 $[g/l]$ | Platin 1,0 $[g/l]$ | Eisen 1,0 $[g/l]$ |
|---|---|---|---|---|---|
| Austauscher dotiert mit | | | | | |
| 2-Methylbuten-2 | $[g]$ | 14,5 | 2,0 | 1,5 | 4,8 |
| 2-Methylbuten-1 | $[g]$ | 3,8 | 0,6 | 0,2 | 1,2 |
| 3-Methylbuten-1 | $[g]$ | 0,5 | - | - | - |
| Methanol | $[g]$ | 9,5 | 2,1 | 1,7 | 3,7 |
| TAME | $[g]$ | - | 23,6 | 24,9 | 18,6 |
| Ausbeute an TAME | $[%]$ | - | 86,0 | 91,0 | 68,0 |

7

## Ansprüche

1. Verfahren zur Herstellung von Alkyl-tert.-alkylethern aus an der Doppelbindung verzweigten Isoolefinen und Alkanolen in Gegenwart von Kationenaustauschern, dadurch gekennzeichnet, daß man zur Veretherung makroporöse oder gelförmige, saure Kationenaustauscher in der H$^+$-Form, die mit 0,1 bis 5 g eines Metalls der VI., VII oder VIII. Nebengruppe des Periodensystems der Elemente (Mendelejew) in elementarer Form pro 1 l trockenen Kationenaustauscher belegt worden sind, benutzt, welche Vernetzungsgrade von 2 bis 65 % und eine spezifische Oberfläche von 5 bis 750 m$^2$ pro g trockenes Austauscherharz aufweisen, wobei das gewünschte Metall in Form eines nicht komplexen, kationischen Salzes mit dem Kationenaustauscher zusammengebracht wird und der Kationenaustauscher nach Neutralwaschen und Trocknen in einer Wasserstoffatmosphäre bei einen Druck von 2-50 bar und einer Temperatur von 50-140 °C behandelt wird, und daß die Veretherungsreaktion in an sich bekannter Weise in der flüssigen Phase bei 30 bis 140 °C, einem Druck von 2 bis 100 bar und einem Molverhältnis von Isoolefin zu Alkanol wie 0,1 bis 5, gegebenenfalls in Gegenwart eines Lösungsmittels, durchgeführt wird.

2. Verfahren nach Anspruch 1, dadurch gekennzeichnet, daß als Metall der VI., VII oder VIII. Nebengruppe Chrom, Rhenium, Palladium, Platin oder Eisen verwendet werden.

3. Verfahren nach Ansprüchen 1 und 2, dadurch gekennzeichnet, daß das Molverhältnis Isoolefin zu Alkanol 0,9 bis 1,1 beträgt.

4. Verfahren nach Ansprüchen 1 bis 3, dadurch gekennzeichnet, daß die Veretherung bei einer Raumgeschwindigkeit von 0,1 bis 10, abläuft.

5. Verfahren nach Ansprüchen 1 bis 4, dadurch gekennzeichnet, daß die Veretherung bei einer Temperatur von 50 bis 130 °C erfolgt.

6. Verfahren nach Ansprüchen 1 bis 5, dadurch gekennzeichnet, daß der Reaktionsdruck im Bereich von 3 bis 30 bar gehalten wird.

7. Verfahren nach Ansprüchen 1 bis 6, dadurch gekennzeichnet, daß als Lösungsmittel gesättigte, verzweigte und/oder unverzweigte Kohlenwasserstoffe mit mindestens 4 Kohlenstoffatomen eingesetzt werden.

8. Verfahren nach Ansprüchen 1 bis 7, dadurch gekennzeichnet, daß zur Veretherung ein Rückführstrom im Gewichtsverhältnis von 0,1 bis 10 : 1, bezogen auf den Einspeisestrom, zusätzlich eingespeist wird.

## Claims

1. Process for the preparation of alkyl tert.-alkyl ethers from isoolefines which are branched at the double bond and from alkanols in the presence of cation exchangers, characterised in that macro-porous or gel-like, acidic cation exchangers in the H$^+$-form, which have been charged with 0.1 to 5 g of a metal of subgroup VI, VII or VIII of the periodic table of the elements (Mendeleev) in the elementary form per litre of dry cation exchanger are used for the etherification, the cation exchangers having degrees of crosslinking of 2 to 65 % and a specific surface area of 5 to 750 m$^2$ per g of dry exchanger resin, the desired metal in the form of a non-complex, cationic salt being brought together with the cation exchanger and the latter, after having been washed until neutral and dried in a hydrogen atmosphere at a pressure of 2-50 bar and a temperature of 50-140 °C, and that the etherification reaction is carried out in a manner known per se in the liquid phase at 30 to 140 °C and a pressure of 2 to 100 bar, using a molar ratio of isoolefine to alkanol of 0.1 to 5, if appropriate in the presence of a solvent.

2. Process according to Claim 1, characterised in that chromium, rhenium, palladium, platinum or iron is used as the metal of sub-group VI, VII or VIII.

3. Process according to Claims 1 and 2, characterised in that the molar ratio of isoolefine to alkanol is 0.9 to 1.1.

4. Process according to Claims 1 to 3, characterised in that the etherification takes place at a space velocity of 0.1 to 10.

5. Process according to Claims 1 to 4, characterised in that the etherification is carried out at a temperature of 50 to 130 °C.

6. Process according to Claims 1 to 5, characterised in that the reaction pressure is kept in the range from 3 to 30 bar.

7. Process according to Claims 1 to 6, characterised in that saturated, branched and/or unbranched hydrocarbons with at least 4 carbon atoms are employed as the solvent.

8. Process according to Claims 1 to 7, characterised in that for the etherification a recycle stream in a weight ratio of 0.1 to 10 : 1, relative to the feed stream, is additionally fed in.

## Revendications

1. Procédé de fabrication d'alcoyl-t-alcoyl-éthers à partir d'isooléfines ramifiées sur la double liaison et d'alcanols en présence d'échangeurs de cations, caractérisé en ce que pour l'éthérification on utilise des échangeurs de cations acides, macroporeux ou sous forme de gels, sous la forme H⁺, qui ont été chargés avec 0,1 à 5 g d'un métal du VIe, VIIe ou VIIIe sous-groupe du système périodique des éléments (Mendeléïev) sous la forme élémentaire par litre d'échangeur de cations sec, qui présentent des degrés de réticulation de 2 à 65 % et une surface spécifique de 5 à 750 m² par g de résine échangeuse sèche, le métal désiré étant réuni sous la forme d'un sel cationique non complexe avec l'échangeur de cations, l'échangeur de cations, après lavage jusqu'à neutralité et séchage, étant traité en atmosphère d'hydrogène sous une pression de 2 à 50 bars et à une température de 50 à 140 °C, et en ce que la réaction d'éthérification est exécutée d'une manière connue en elle-même en phase liquide à 30-140 °C, sous une pression de 2 à 100 bars et à un rapport molaire de l'isooléfine envers l'alcanol de 0,1 à 5, éventuellement en présence d'un solvant.

2. Procédé selon la revendication 1, caractérisé en ce qu'on utilise comme métal du VIe, VIIe ou VIIIe sous-groupe du chrome, du rhénium, du palladium, du platine ou du fer.

3. Procédé selon les revendications 1 et 2, caractérisé en ce que le rapport molaire entre isooléfine et alcanol s'élève à 0,9-1,1.

4. Procédé selon les revendications 1 à 3, caractérisé en ce que l'éthérification s'effectue à une vitesse spatiale de 0,1 à 10.

5. Procédé selon les revendications 1 à 4, caractérisé en ce que l'éthérification s'effectue à une température de 50 à 130 °C.

6. Procédé selon les revendications 1 à 5, caractérisé en ce que la pression de réaction est maintenue dans l'intervalle de 3 à 30 bars.

7. Procédé selon les revendications 1 à 6, caractérisé en ce qu'on utilise comme solvants des hydrocarbures saturés, ramifiés et/ou non ramifiés, ayant au moins 4 atomes de carbone.

8. Procédé selon les revendications 1 à 7, caractérisé en ce que pour l'éthérification on alimente supplémentairement un courant de recyclage dans le rapport pondéral de 0,1 à 10 : 1 envers le courant d'alimentation.